(19) Europäisches Patentamt
European Patent Office
Office européen des brevets

(11) Publication number: **0 186 436 B1**

# (12) EUROPEAN PATENT SPECIFICATION

(45) Date of publication of patent specification: **18.03.92**

(51) Int. Cl.⁵: **A61K 7/30**, C11D 10/02

(21) Application number: **85309247.6**

(22) Date of filing: **18.12.85**

## (54) Liquid cleansing composition.

(30) Priority: **20.12.84 US 684818**

(43) Date of publication of application:
**02.07.86 Bulletin 86/27**

(45) Publication of the grant of the patent:
**18.03.92 Bulletin 92/12**

(84) Designated Contracting States:
**AT BE CH DE FR GB IT LI LU NL SE**

(56) References cited:
**EP-A- 0 117 889**
**DE-A- 3 206 350**

(73) Proprietor: **WARNER-LAMBERT COMPANY**
**201 Tabor Road**
**Morris Plains New Jersey 07950(US)**

(72) Inventor: **Eoga, Anthony B.J.**
**321 Rexland Drive**
**Boonton New Jersey 07005(US)**

(74) Representative: **Jones, Michael Raymond et al**
**HASELTINE LAKE & CO. Hazlitt House 28**
**Southampton Buildings Chancery Lane**
**London WC2A 1AT(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to a liquid cleanser composition suitable for cleaning dentures and designed to be delivered through a spray pump system or device.

The denture cleanser prior art has focused on the use of oxidizing agents and bleaching agents in concert to remove visible stain and scale or plaque buildup. For the most part these compositions have employed a variety of sulfate salts, e.g., bisulfates and monopersulfates, to serve as detergents and oxidizers, as well as alkali metal and alkali earth metal halides as bleaches. Perborate, carbonate, bicarbonate and phosphate salts were conventionally included to provide effervescence and activation. These references have focused on making powdered or tableted products which dissolve rapidly and effervesce when placed in water. These products clean dentures generally in 12-30 minutes or longer and require constant emersion during this period, followed by brushing and rinsing with water.

The persulfate and perborate systems of the prior art tend to discolor the stain on the denture. Tablets made from these ingredients were limited to small amounts of detergents or surfactants in order to achieve fast dissolution and subsequent cleansing when placed in water. Detergents of the high fatty alcohol type could therefore not be incorporated except in minute quantities. e.g., 1% or less, or poor tablet dissolution rates resulted. The use of hypochlorites as bleaching agents discolor denture stain to an even greater extent than the persulfates and perborates. These chlorinated agents have a more serious disadvantage in that they produce an intolerable and unpleasant after taste and odor and may adversely discolor denture metal under certain conditions.

Additionally, although the prior art powder and tablet systems remove some of the denture stain, they tend to leave an undissolved residue which can only be removed mechanically. Consumers usually revert to a soaking and brushing regimen to obtain a thoroughly clean denture. Other types of undissolved residue include chewing gum residue and denture adhesive residue. These residues are particularly difficult to remove even with a regular brushing regimen since they became imbedded in the dentures or partial dentures. This obviously has adverse effects on the denture wearer. Even those chewing gum having non-stick properties and specially formulated for denture wearers become to some extent imbedded in the denture crevices. Denture wearers generally prefer a strong denture adhesive to insure proper adhesive of the denture in the mouth. These types of adhesives have the disadvantage of being very difficult to remove and clean from the denture. The use of water in combination with denture cleanser pastes tend to spread out rather than remove the chewing gum and adhesive residue and therefore fail to remove them from the denture surface. The inventive compositions solve this problem by loosening the chewing gum and adhesive residue such that it can then be removed with gentile rubbing with the fingers.

More recently a liquid denture cleanser has been disclosed in South African Patent Application No. 83/4286.) This reference discloses a foam-producing liquid composition comprising from about 1 to about 10% by weight of a pharmaceutically acceptable surfactant, about 35 to about 70% by weight of ethanol or isopropanol or mixtures thereof, about 0.1 to about 10% by weight of a pharmaceutically acceptable humectant, about 25 to about 60% water and about 0-5% of an adjuvant. The composition must not contain more than 50% water when 42% alcohol is present and vice versa. Those surfactants disclosed are sodium lauryl sulfate, sodium dodecyl benzene sulfonates, polysorbate 80, poloxomer 407 and mixtures thereof. Humectants are selected from the group consisting of glycerin, sorbitol, polyethylene glycol, propylene glycol and mixtures thereof. The use of the trisodium salt of ethylene diamine tetracetic acid (EDTA) in an amount of about 0.5% is also disclosed a useful adjuvant.

Figures I and II are photographs of stained dentures. These pictures clearly demonstrate the superior cleaning efficacy of the inventive liquid spray denture compositions over the prior art tableted compositions as well as the prior art liquid denture cleanser of South African Patent Application No. 83/4286 described above.

In DE-A-3206350, there is disclosed a liquid detergent composition, intended for use mainly as a shampoo composition, which comprises:

(A) 5 to 25 weight % of an ammonium alkyl sulphate of a given general formula;

(B) 0.05 to 5 weight % of an alkylenediaminecarboxylic acid derivative of a given general formula; and

(C) 0.1 to 5 weight % of a carboxylic acid, a salt of a carboxylic acid, an amino acid, or salt of an amino acid.

According to the present invention, there is provided a liquid cleanser composition, suitable for cleansing dentures, comprising:

(i) a sulfated or sulfonated fatty alcohol salt detergent which includes a $C_{10-16}$ alkyl moiety and an alkali or alkaline earth metal, and which optionally includes an aromatic moiety, the detergent optionally having the formula $ROSO_3M$, wherein R is a $C_{10-16}$ alkyl and M is a water soluble alkali metal, the detergent

being present in the composition in an amount of from 10% to 15%, by weight of the total composition;

(ii) a chelating agent of the amino carboxylate or organo phosphonate type present in the composition in an amount of from 3% to 12%, by weight of the total composition; and

(iii) from 50% to 87% of water.

The composition of the present invention does not contain any of the component (C) as is present in the composition of DE-A-3206350.

The liquid compositions of the present invention are designed to be dispensed from a pump spray bottle. The compositions do not use oxidizing agents or chlorine-containing compounds and consequently do not suffer from the disadvantages associated with these agents, as does the prior art. The liquid cleanser is sprayed onto the denture, allowed to stand for about 20 to about 120 seconds and rinsed in tap water. The length of time the cleanser is permitted to remain on the denture is dictated by the type and intensity of stain. Most food, tobacco and plaque stains, however, are easily removed after about 2 minutes of contact with the liquid cleanser. The force of the pump spray supplements the cleansing effect of the cleanser. The pump spray force is designed to maximize the removal of food particles and stain on the denture surface. Thus, a minimum spray force is required such that is provides a mechanical cleansing action without a separate brushing step. The maximum force must be kept within a range which is controllable and convenient for the user. This is dictated primarily by consumer acceptability and marketing considerations.

The amounts of detergent and chelating agent used are sufficient to remove stain and plaque and to eliminate malodor in the denture.

The weight ratio of detergent to chelating agent is preferably about 1:1 to about 2:1.

The use of sulfated or sulfonated higher fatty alcohols as detergents is well known. Such anionic detergents, however, when mixed with a suitable chelating agent, have surprisingly provided a highly effective denture cleanser which, when applied with the application force of a pump spray, provided a more convenient and efficient denture cleanser than the prior art methods. The user is able to clean his dentures without soaking them, as required in the prior art tablet and powder compositions.

It is critical to the invention that the sulfated or sulfonated fatty alcohol detergent be used in conjunction with the recited chelating agents in aqueous solution. These detergents when used by themselves and dispensed from a mechanical spraying device or from an aerosol mixture, produce an intolerable situation which is harmful to the respiratory tract. Uncontrollable sneezing or coughing and respiratory irritation results. Such a situation would restrict the use to well ventilated, controlled areas. It has been discovered, quite unexpectedly, that these detergents can be atomized or sprayed without these harmful side effects, by forming an aqueous mixture of the detergent with an amino carboxylate or organo phosphonate chelating agent. This combination both alleviates the adverse respiratory and inhalation problems associated with the detergents and increases the cleaning efficacy of the composition. The weight ratio of detergent to chelator recited above has been carefully selected to achieve these results.

Examples of useful sulfated or sulfonated fatty alcohol detergents are selected from the anionic water soluble class. The potassium and sodium salts of the higher alkyl benzene sulfonates are preferred. Among these, the sodium linear alkyl benezene sulfonates are preferred. Other examples include magnesium lauryl sulfate, potassium lauryl sulfate, sodium dodecylbenzene sulfonate, sodium lauryl sulfoacetate, sodium myristyl sulfate, sodium lauryl sulfate, sodium cetyl sulfate, sodium tridecyl sulfate and sodium-7-ethyl-2-methyl-4-undecyl sulfate. Mixtures of any and all of these detergents are also useful.

Other anionic detergents may be incorporated into the composition along with the above required detergents. These supplemental detergents may be present in amounts of about 1 to about 5%. These additional detergents include compounds such as the oleic acid ester of sodium isethionate, sodium N-cyclohexyl-N-palmitoyl taurate, sodium N-coconut acid-N-methyl taurate, sodium N-methyl-N-oleyl taurate and mixtures thereof. Fluorochemical surfactants, commonly known as Fluorads®, are also useful additives. Examples include those compounds disclosed in British Patent 1,322,548, having the formula:

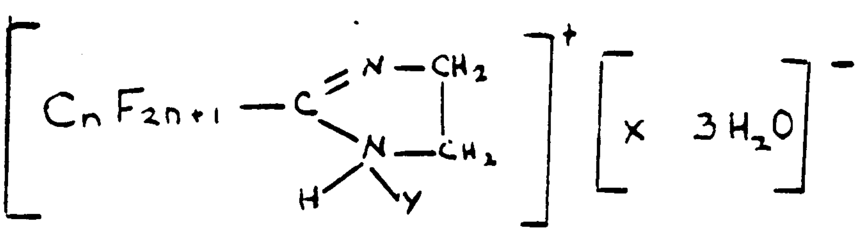

wherein n is an integer from 3 to 10, X is bromine or iodine; and Y is a glycol residue having 3 to 10 carbon atoms derived, for example, from ethylene glycol or propylene glycol as an ethyoxylated alcohol having 1 to 10 moles of ethylene or propylene oxide.

The use of chelating or sequestering agents in the prior art was typically in combination with denture cleanser tablets containing peroxygen bleaching agents. The tetrasodium salt of ethylenediamine tetracetic acid, among others, was commonly used to sequester the heavy metal impurities present in solution and prevent the decomposition of the bleaching agent.

Useful amino carboxylate chelators include tetrasodium ethylenediaminetetracetate dihydrate, trisodium ethylenediaminetetracetate, disodium ethylenediaminetetracetate dihydrate, trisodium N-hydroxyethlyethylenediaminetriacetate hydrate, trisodium nitrilotriacetate monohydrate, pentasodium diethylenetriaminepentaacetate, trisodium ethylenediaminetetracetate trihydrate and mixtures thereof. These chelators, as well as other well known amino carboxylates are generally used in amounts sufficient to clean stain and plaque when combined with the detergent. They are used in amounts of from 3 to 12% by weight and more preferably about 5 to about 10%.

Those organic phosphates useful may be selected from a wide range of conventional materials. Examples include 1-hydroxy-1,1-diphosphonic acid and its sodium and potassium salts. These salts are also sold under the tradename Dequest by Monsanto Company. In addition to being chelating agents, the organic phosphonates serve as detergent builders. These chelating agents are employed in the same amounts as the amino carboxylates. Mixtures of any and all of the chelators is also contemplated.

The inventive liquid denture cleansers can optionally contain detergent builders which are conventionally used in detergent formulations and which enhance cleaning and contribute to a brighter surface appearance of the denture. Useful builders include the water-soluble phosphates, pyrophosphates, ortho phosphates, carbonates and the like. Specific examples of inorganic phosphate builders useful include the sodium or potassium salts of tripolyphosphates, pyrophosphates and hexametaphosphates. As mentioned above, the organic phosphonate chelating agents also serve as builders. When incorporated into the composition, builders are generally present in amounts of 3% to 12%: and preferably in amounts of about 5% to about 10%.

Other conventional additives such as flavorings, colorants, perfumes, antimicrobials, preservatives, and the like may be optionally incorporated in the inventive compositions. For example, flavorings such as mint, oil of clove, artificial vanilla, to name a few, are useful. These materials may be blended in various combinations and the amounts may be varied according to the perception desired.

In the instance where colorants are used, F.D.& C. and D.& C. dyes may be used. Lakes are generally not useful due to their insolubility in the inventive compositions. Those dyes useful are certified by the Federal Food & Drug Administration as acceptable for use in food, drug and cosmetic applications and in drug and cosmetic colorings. The materials acceptable for the foregoing are preferably water-soluble and include F.D. & C. Blue No. 2 (indigo dye), F.D. & C. Green No. 1 and 3, to name a few. Conventional preservatives such as methyl and propyl paraben and mixtures thereof may be incorporated in amounts well known in the art.

As previously mentioned, the inventive liquid denture, cleanser compositions are designed to be applied to the denture surface from a mechanical spraying device. Simple hand spray pump bottles capable of disposing high viscosity fluids are sufficient. Aerosols, including piston-driven types, are also useful, but are not preferred since they are more costly and do not afford any advantages over the mechanical devices for purposes of this invention. Application by spraying imparts a certain mechanical cleaning force as the liquid hits the denture surface. By directing the spraying parameters, e.g., the spray pattern, the weight of liquid applied and the speed of the spray, the cleaning force can be controlled.

In measuring the force, one fluid ounce propylene cylindrical pump bottles having a high viscosity spray dispensing head were filled with the inventive compositions. The denture or other target was held stationery in an Instron tensile testing machine. The spray bottle was mounted horizontally above the target with the spray nozzle at a distance of 1" (2.54 cm) away. An air driven piston/solenoid device was used to actuate the spray pump. The air pressure driving the piston was approximately 138000 $N/m^2$ (5 psi). The peak force of the spray against the target was recorded by a microprocessor connected to the Instron load cell's output. The measurement was in gram force units. The results indicated that a force of at least $9.8 \times 10^{-3}$ (1 gram force) and preferably between about $9.8 \times 10^{-3}$ to $29.4 \times 10^{-3}$N (1 to 3 gram force) is required to provide enough mechanical action to supplement the cleaning effect of the composition itself.

The weight of the liquid dispensed per actuation of the mechanical spraying device was measured by subtracting the weight of the liquid and spray bottle after dispensation with the weight prior to dispensation. The average weight delivered per actuation is about 150 to about 200 mg of liquid; and preferably about 160 to about 180 mg ± 25 mg of liquid. These amounts are preferred for adequate cleaning of most stains

and plaque.

The spray pattern was determined by stationing the spray pump bottle and directing the spray nozzle onto an absorbant pad situated about 2" (5.1cm) away. The pump sprayer was actuated once and the vertical and horizontal diameters of the spray pattern were measured as they appeared on the pad. The spray pattern is preferably about 3.8 cm (1.5") to about 6.35 cm (2.5") by 3.8 cm (1.5") to about 6.35 cm (2.5") at a distance of about 5.1 cm (2") from the target. Most preferably, the spray pattern is about 4.7 ± 1.7 cm (1.85" ± 0.65") x 4.7 ± 1.7 cm (1.85 ± 0.65") at the 5.1 cm (2") distance from the target.

The above measurements were taken to obtain the minimum parameters related to the mechanical force required to achieve cleaning. While the composition itself is a good cleaner, surprisingly improved cleaning of the denture is achieved when the composition is sprayed with sufficient mechanical force to help remove stain, plaque and other debris.

Additional considerations must be given to the choice of detergent/chelator formulation such that the viscosity is not so high that dispensing from a spray bottle is difficult. For example, when ethylenediamine tetracetic tetrasodium salt is used in conjunction with sodium lauryl sulfoacetate (Lanthanol LAL) or another linear alkyl sulfonate, the detergent tends to salt out, becoming a thick concentrate and making spraying difficult. Thus, it is important that the chelators be water-soluble and that the combination of the chelator and detergent result in a viscosity which is readily and easily dispensed from a mechanical spraying device. The viscosity problems and the salting-out effect generally occur at concentrations of chelator above 5% and at temperatures below room temperature. To help alleviate these problems, agents which depress the freezing point can be added. Such agents include various glycol mixtures such as carbowax 400 (polyethylene glycol having a molecular weight of about 380-420), and are present in amounts of about 0.5 to about 2% by weight, and preferably about 0.8 to about 1%.

The term "denture" as used herein, includes artificial teeth, removable orthodontic bridges, dentures, plates and the like.

A high degree of cleansing is generally achieved in less than 1 minute and preferably in 30 seconds or less. The cleansers, with the aid of the mechanical force exerted by the spray, strip off plaque, remove mucous resulting from the deposition of proteins and glycoproteins, remove food particles and debris, remove and/or kill bacteria, remove residual denture adhesives and chewing gum, remove food stains and tobacco stains as well as coffee and tea stains. These compositions have been shown to effectively loosen chewing gum and denture adhesive such that these residues can be easily removed by gently rubbing with the fingers or gentle brushing. These cleansing effects are achieved without adversely affecting the metal, plastic or other materials of the denture construction.

The instant invention allows the denture wearer to clean his dentures quickly, conveniently in a portable package without the need for a container for soaking.

A fuller understanding of the present invention will be gained from the following illustrative examples. Unless specified otherwise all amounts expressed as percent are intended to be a percent by weight of the total composition.

Trade names and registered trade marks are acknowledged as such.

## EXAMPLE ONE

Denture Cleanser compositions were prepared using the formulations tabulated below. Composition A represents a preferred embodiment of the inventive liquid denture spray composition. Compositions B and C represent a commercial denture tablets.

| Ingredients | Composition | | |
|---|---|---|---|
| | A (Inventive) | B (Prior art tablet) | C (Prior art tablet) |
| Sodium Lauryl Sulfate | 10% | - | - |
| Ethylenediaminetetraacetic Acid Tetrasodium Salt | 5% | 3.4 | 1.25 |
| Flavor, color, preservative | 0.5% | - | - |
| Water | 85.5% | - | - |
| Sodium Bicarbonate | - | - | 14.0 |
| Citric Acid | - | - | 10.3 |
| Sodium Carbonate | - | | 12.8 |
| Colorant | - | 0.2 | 0.1 |
| Oxone | - | - | 39.5 |
| Flavor and Fragrance | - | 0.5 | 1.0 |
| Detergent | - | 0.5 | 0.65 |
| Magnesium Stearate | | | 0.2 |
| Sodium Perborate Monohydrate | - | 37.3* | 12.5 |
| Anhydrous Sodium Perborate | - | 22.85* | - |
| Trisodium Phosphate | - | 33.8 | - |
| Sodium Benzoate | - | 1.0 | 1.6 |
| Polytetraflouroethylene | - | 0.45 | - |
| Filler | - | - | 6.1 |

* Includes approximately 0.45% by weight of total perborate polytetrafluoroethylene prepared as a granulated mixture.

In vitro denture tiles were prepared with plaque, a composite of food stain consisting of grapes, blueberry, tea and coffee as well as tobacco stain.

Samples of the tiles were then chosen to be cleansed with one of the three compositions listed above. The treatment with Composition A (inventive) was accomplished by spraying the tiles and allowing the spray to remain on each tile type for 30, 60 and 120 seconds prior to rinsing with water at 45° C for 20 seconds.

The treatment with the tablets of Composition B and C (prior art) was accomplished by dropping the tablets in beakers of 45° C water containing the tiles. Those tiles treated with Composition B were allowed to soak for 5 minutes. Those tiles treated with Composition C were allowed to soak for 12 minutes.

The results showed that tiles cleaned by the inventive Composition A for 120 seconds were significantly cleaner than those cleaned with Compositions B and C. The tiles cleaned with Composition A were visibly cleaner than those cleaned with B and C.

Those denture tiles which were cleansed with the inventive Composition A for 30 and 60 seconds required an additional spraying and an immediate rinsing in order to achieve similar results to the 120 second treatment.

Those tiles which had 24 hours plaque accumulated on them were sprayed with Composition A and allowed to stand for 2 minutes. Additional plaque tiles were soaked in Compositions B and C for 12 and 5 minutes respectfully. All the tiles were then rinsed under running water at 45° C for 20 seconds and immersed in a basic 0.01% Fuchsin solution. The Fuchsin solution is used as an indicator for plaque. The tiles were then rinsed again and qualitatively compared for red color. The tiles cleaned with Composition A (inventive) had the least amount of red color resulting form the Fuchsin, and consequently the least amount of plaque.

EXAMPLE TWO

This example is intended to show a comparison between the cleansing efficacy of inventive compositions as compared to a well known commercial tablet of the prior art and the liquid foaming denture cleanser disclosed in South African Application No. 83/4286, page 11, composition C.

A set of tiles (Set I) were prepared with coffee, tea, blueberry and grape stains. Another set (Set II) of tiles were prepared having tobacco, coffee, tea, blueberry and grape on a plaque matrix.

Figure I shows the results of the food stained tiles after cleaning with each of the denture cleansers. "Prior Art I," as labeled in the Figures, is the commercial tableted denture cleanser, which was dissolved in a beaker of water at 45° C containing the tile and left for 12 minutes.

The "Inventive Composition," as labeled in the Figures, is formulation A set forth in Example 1. The

spray was left on the tile for 120 seconds and rinsed with water for 20 seconds at 45 °C.

"Prior Art II," as labeled in the Figures, is the liquid foaming Composition C set forth on page 11 of South African Application No. 83/4286. The chelator EDTA has been added for the sake of comparison to the instant inventive compositions. This composition had the following formula:

| | |
|---|---|
| Sodium dodecyl benzene sulfonate | 3% |
| Polysorbate 80 | 1% |
| Glycerin | 1.5% |
| PEG 400 | 0.5% |
| Ethyl alcohol | 55% |
| Water | 36.75% |
| Flavor/color | 1.75% |
| EDTA | 0.5% |

From observing the photographs in Figures I and II, the cleaner (whiter) surfaces are obviously those cleansed with the inventive compositions. It is apparent that the inventive liquid spray denture cleanser compositions exhibited significantly better cleaning than the prior art tableted denture cleanser and the prior art liquid denture cleansers.

In the sulfated or sulfonated fatty alcohol detergent salt, the $C_{10-16}$ alkyl moiety may be part of an alkaryl or aralkyl group when the detergent salt also contains an aromatic moiety.

**Claims**

**Claims for the following Contracting States : BE, CH, DE, FR, GB, IT, LI, LU, NL, SE**

**1.** A liquid cleanser composition, suitable for cleansing dentures, comprising:
(i) a sulfated or sulfonated fatty alcohol salt detergent which includes a $C_{10-16}$ alkyl moiety and an alkali or alkaline earth metal, and which optionally includes an aromatic moiety, the detergent optionally having the formula $ROSO_3M$, wherein R is a $C_{10-16}$ alkyl and M is a water soluble alkali metal, the detergent being present in the composition in an amount of from 10% to 15%, by weight of the total composition;
(ii) a chelating agent of the amino carboxylate or organo phosphonate type present in the composition in an amount of from 3% to 12%, by weight of the total composition; and
(iii) from 50% to 87% of water;
wherein the composition does not contain any of carboxylic acid, salt of carboxylic acid, amino acid, or salt of amino acid.

**2.** A composition according to Claim 1, wherein the sulfated fatty alcohol detergent is selected from potassium lauryl sulfate, magnesium lauryl sulfate, sodium lauryl sulfate, sodium cetyl sulfate, sodium tridecyl sulfate, sodium-7-ethyl-2-methyl-4-undecyl sulfate, sodium dodecylbenzene sulfonate, sodium lauryl sulfonacetate and mixtures thereof.

**3.** A composition according to Claim 1 or 2, wherein the chelating agent is selected from tetrasodium ethylenediaminetetraacetate dihydrate, trisodium ethylenediaminetetraacetate, sodium ethylenediaminetetraacetate dihydrate, trisodium N-hydroxyethylenediaminetriacetate hydrate, trisodium nitrilotriacetate monohydrate, pentasodium diethylenetriaminepentaacetate, trisodium ethylenediaminetetraacetate trihydrate, the sodium salt of 1-hydroxy-1,1-diphosphonic acid, the potassium salt of 1-hydroxy-1,1-diphosphonic acid and mixtures thereof.

**4.** A composition according to Claim 1, 2 or 3, wherein the range of detergent to chelating agent is in the ratio of 1:1 to 2:1.

**5.** A composition according to any preceding claim, wherein there is additionally present a material selected from additional anionic detergents, freezing point depressants, flavourings, colourings, perfumes, preservatives, builders, antimicrobials and mixtures thereof.

**6.** A composition according to Claim 5, wherein the preservative is a paraben; or wherein the flavouring is mint; or wherein the builder is selected from water-soluble, phosphates, pyrophosphates, ortho

phosphates, carbonates and mixtures thereof; or wherein the colouring is an F.D. & C. or D. & C. dye; or wherein the freezing point depressant is a glycol mixture in an amount of 0.5 to 2%; or wherein the additional anionic detergent is selected from the oleic acid ester of sodium isethionate, sodium N-cyclohexyl-N-palmitoyl taurate, sodium N-coconut acid-N-methyl taurate, sodium N-methyl-N-oleyl taurate, fluorochemical surfactants having the formula:

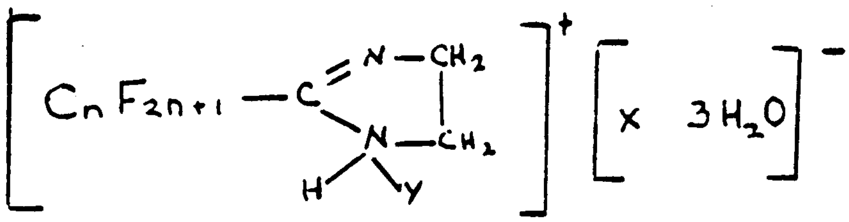

wherein n is an integer from 3 to 10, X is bromine or iodine, and Y is a glycol residue having 3 to 10 carbon atoms derived from, for example, ethylene glycol or propylene glycol as an ethyoxylated alohol having 1 to 10 moles of ethylene or propylene oxide, and mixtures thereof.

**7.** A method of cleaning a denture, comprising:
(a) spraying the denture with a liquid cleanser composition as claimed in any of claims 1 to 6; and
(b) rinsing the denture with water.

**8.** A method according to Claim 7, wherein the spray has a force of at least $9.8 \times 10^{-3}$N (one gram force); or wherein the spray has a pattern with at least 3.8 cm (1.5 inch) to 6.35 cm (2.5 inch) when sprayed from a distance of 5.1 cm (2 inch) from the denture; or wherein the liquid denture composition is allowed to remain on the denture for from 20 to 120 seconds prior to rinsing with water.

**Claims for the following Contracting State : AT**

**1.** A process for producing a liquid cleanser composition, suitable for cleansing dentures, which comprises admixing:
(i) a sulfated or sulfonated fatty alcohol salt detergent which includes a $C_{10-16}$ alkyl moiety and an alkali or alkaline earth metal, and which optionally includes an aromatic moiety, the detergent optionally having the formula $ROSO_3M$, wherein R is a $C_{10-16}$ alkyl and M is a water soluble alkali metal, the detergent being present in the composition in an amount of from 10% to 15%, by weight of the total composition;
(ii) a chelating agent of the amino carboxylate or organo phosphonate type present in the composition in an amount of from 3% to 12%, by weight of the total composition; and
(iii) from 50% to 87% of water;
wherein the composition does not contain any of carboxylic acid, salt of carboxylic acid, amino acid, or salt of amino acid.

**2.** A process according to Claim 1, wherein the sulfated fatty alcohol detergent is selected from potassium lauryl sulfate, magnesium lauryl sulfate, sodium lauryl sulfate, sodium cetyl sulfate, sodium tridecyl sulfate, sodium-7-ethyl-2-methyl-4-undecyl sulfate, sodium dodecylbenzene sulfonate, sodium lauryl sulfonacetate and mixtures thereof.

**3.** A process according to Claim 1 or 2, wherein the chelating agent is selected from tetrasodium ethylenediaminetetraacetate dihydrate, trisodium ethylenediaminetetraacetate, sodium ethylenediaminetetraacetate dihydrate, trisodium N-hydroxyethylenediaminetriacetate hydrate, trisodium nitrilotriacetate monohydrate, pentasodium diethylenetriaminepentaacetate, trisodium ethylenediaminetetraacetate trihydrate, the sodium salt of 1-hydroxy-1,1-diphosphonic acid, the potassium salt of 1-hydroxy-1,1-diphosphonic acid and mixtures thereof.

**4.** A process according to Claim 1, 2 or 3, wherein the ratio of detergent to chelating agent is in the range of 1:1 to 2:1.

5. A process according to any preceding claim, wherein there is additionally present a material selected from additional anionic detergents, freezing point depressants, flavourings, colourings, perfumes, preservatives, builders, antimicrobials and mixtures thereof.

6. A process according to Claim 5, wherein the preservative is a paraben; or wherein the flavouring is mint; or wherein the builder is selected from water-soluble, phosphates, pyrophosphates, ortho phosphates, carbonates and mixtures thereof; or wherein the colouring is an F.D. & C. or D. & C. dye; or the freezing point depressant is a glycol mixture in an amount of 0.5 to 2%; or wherein the additional anionic detergent is selected from the oleic acid ester of sodium isethionate, sodium N-cyclohexyl-N-palmitoyl taurate, sodium N-coconut acid-N-methyl taurate, sodium N-methyl-N-oleyl taurate, fluorochemical surfactants having the formula:

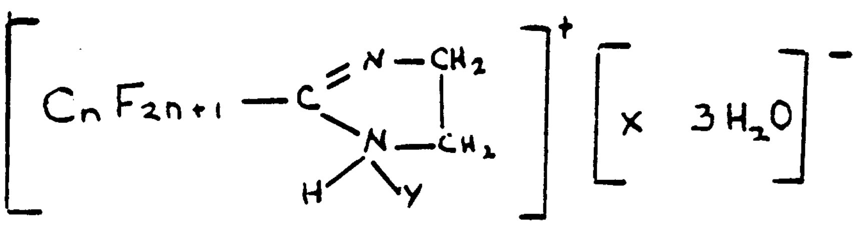

wherein n is an integer from 3 to 10, X is bromine or iodine, and Y is a glycol residue having 3 to 10 carbon atoms derived from, for example, ethylene glycol or propylene glycol as an ethyoxylated alcohol having 1 to 10 moles of ethylene or propylene oxide, and mixtures thereof.

7. A method of cleaning a denture, comprising:
(a) spraying the denture with a liquid cleanser composition produced by a process according to any of claims 1 to 6; and
(b) rinsing the denture with water.

8. A method according to Claim 7, wherein the spray has a force of at least 9.8 x $10^{-3}$N (one gram force); or wherein the spray has a pattern with at least 3.8 cm (1.5 inch) to 6.35 cm (2.5 inch) when sprayed from a distance of 5.1 cm (2 inch) from the denture; or wherein the liquid denture composition is allowed to remain on the denture for from 20 to 120 seconds prior to rinsing with water.

**Revendications**
**Revendications pour les Etats contractants suivants : BE, CH, DE, FR, GB, IT, LI, LU, NL, SE**

1. Une composition nettoyante liquide, convenant au nettoyage des prothèses dentaires, comprenant:
(i) un détergent constitué d'un sel d'un alcool gras sulfaté ou sulfoné, qui comprend un fragment alkyle en $C_{10-16}$ et un métal alcalin ou alcalino-terreux, et qui éventuellement contient un fragment aromatique, le détergeant ayant éventuellement la formule $ROSO_3M$, dans laquelle R est un radical alkyle en $C_{10-16}$ et M est un métal alcalin soluble dans l'eau, le détergeant étant présent dans la composition en une quantité de 10 à 15% en poids par rapport à la composition totale;
(ii) un agent chélatant du type aminocarboxylate ou organophosphonate, présent dans la composition en une quantité de 3 à 12% en poids par rapport à la composition totale; et
(iii) de 50 à 87% en poids d'eau;
où la composition ne contient ni acide carboxylique, ni aucun sel d'acide carboxylique, ni acide aminé, ni aucun sel d'acide aminé.

2. Une composition selon la revendication 1, dans laquelle le détergent à base d'un alcool gras sulfaté est choisi parmi le laurylsulfate de potassium, le laurylsulfate de magnésium, le laurylsulfate de sodium, le cétylsulfate de sodium, le tridécylsulfate de sodium, le 7-éthyl-2-méthyl-4-undécylsulfate de sodium, le dodécylbenzènesulfonate de sodium, le laurylsulfone-acétate de sodium et leurs mélanges.

3. Une composition selon la revendication 1 ou 2, dans laquelle l'agent chélatant est choisi parmi l'éthylènediaminetétraacétate tétrasodique dihydraté, l'étylènediaminetétraacétate trisodique, l'éthylènediaminetétraacétate de sodium dihydraté, le N-hydroxyéthylènediaminetriacétate trisodique hydraté, le

nitrilotriacétate trisodique monohydraté, le diéthylènetriaminepentaacétate pentasodique, l'éthylènediaminetétraacétate trisodique trihydraté, le sel de sodium de l'acide 1-hydroxy-1,1-diphosphonique, le sel de potassium de l'acide 1-hydroxy-1,1-diphosphonique et leurs mélanges.

**4.** Une composition selon la revendication 1, 2 ou 3, dans laquelle le rapport du détergeant à l'agent chélatant est compris entre 1/1 et 2/1.

**5.** Une composition selon l'une quelconque des revendications précédentes, dans laquelle on a, en outre, un produit choisi parmi des détergents anioniques supplémentaires, des abaisseurs de point de congélation, des aromatisants, des colorants, des parfums, des conservateurs, des adjuvants, des agents anti-microbiens et leurs mélanges.

**6.** Une composition selon la revendication 5, dans laquelle le conservateur est un parabène; ou dans laquelle l'aromatisant est la menthe; ou dans laquelle l'adjuvant est choisi parmi les phosphates, pyrophosphates, orthophosphates, carbonates solubles dans l'eau et leurs mélanges; ou dans laquelle le colorant est un colorant F.D. & C. ou D. & C.; ou dans laquelle l'abaisseur de point de congélation est un mélange de glycols en une quantité de 0,5 à 2%; ou dans laquelle le détergent anionique supplémentaire est choisi parmi l'ester de l'acide oléique de l'iséthionate de sodium, le N-cyclohexyl-N-palmitoyl-taurate de sodium, le N-(dérivé acide de l'huile de copra)-N-méthyl-taurate de sodium, le N-méthyl-N-oléyl-taurate de sodium, les tensio-actifs fluorochimiques ayant la formule:

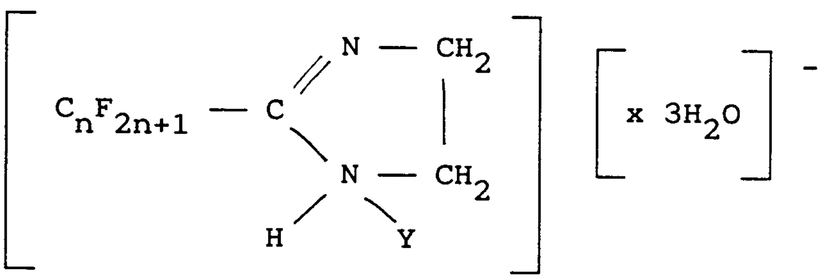

dans laquelle:

- n  est un entier de 3 à 10;
- X  est le brome ou l'iode; et
- Y  est un résidu glycol ayant 3 à 10 atomes de carbone et dérivant par exemple de l'éthylèneglycol ou du propylèneglycol, sous forme d'un alcool éthoxylé ayant 1 à 10 moles d'oxyde d'éthylène ou de propylène,

et leurs mélanges.

**7.** Un procédé de nettoyage d'une prothèse dentaire, comprenant les étapes consistant à:
(a) pulvériser sur la prothèse dentaire une composition nettoyante liquide selon l'une quelconque des revendications 1 à 6; et
(b) rincer la prothèse dentaire à l'eau.

**8.** Un procédé selon la revendication 7, dans laquelle la pulvérisation se fait à une force d'au moins $9{,}8.10^{-3}$ N (un gramme force); ou dans laquelle le jet de pulvérisation est appliqué sur une zone d'au moins 3,8 à 6,35 cm (1,5 à 2,5 inch) quand le jet est pulvérisé à partir d'une distance de 5,1 cm (2 inch) de la prothèse dentaire; ou dans laquelle la composition nettoyante liquide pour prothèse dentaire peut rester sur la prothèse dentaire pendant 20 à 120 secondes avant rinçage à l'eau.

**Revendications pour l'Etat contractant suivant : AT**

**1.** Un procédé de production d'une composition nettoyante liquide, convenant au nettoyage des prothèses dentaires, qui consiste à mélanger:
(i) un détergent constitué d'un sel d'un alcool gras sulfaté ou sulfoné, qui comprend un fragment alkyle en $C_{10-16}$ et un métal alcalin ou alcalino-terreux, et qui éventuellement contient un fragment aromatique, le détergeant ayant éventuellement la formule $ROSO_3M$, dans laquelle R est un radical

alkyle en $C_{10-16}$ et M est un métal alcalin soluble dans l'eau, le détergeant étant présent dans la composition en une quantité de 10 à 15% en poids par rapport à la composition totale;
(ii) un agent chélatant du type aminocarboxylate ou organophosphonate, présent dans la composition en une quantité de 3 à 12% en poids par rapport à la composition totale; et
(iii) de 50 à 87% en poids d'eau;
où la composition ne contient ni acide carboxylique, ni aucun sel d'acide carboxylique, ni acide aminé, ni aucun sel d'acide aminé.

**2.** Un procédé selon la revendication 1, dans lequel le détergent à base d'un alcool gras sulfaté est choisi parmi le laurylsulfate de potassium, le laurylsulfate de magnésium, le laurylsulfate de sodium, le cétylsulfate de sodium, le tridécylsulfate de sodium, le 7-éthyl-2-méthyl-4-undécylsulfate de sodium, le dodécylbenzènesulfonate de sodium, le laurylsulfone-acétate de sodium et leurs mélanges.

**3.** Un procédé selon la revendication 1 ou 2, dans lequel l'agent chélatant est choisi parmi l'éthylènediaminetétraacétate tétrasodique dihydraté, l'étylènediaminetétraacétate trisodique, l'éthylènediaminetétraacétate de sodium dihydraté, le N-hydroxyéthylènediaminetriacétate trisodique hydraté, le nitrilotriacétate trisodique monohydraté, le diéthylènetriaminepentaacétate pentasodique, l'éthylènediaminetétraacétate trisodique trihydraté, le sel de sodium de l'acide 1-hydroxy-1,1-diphosphonique, le sel de potassium de l'acide 1-hydroxy-1,1-diphosphonique et leurs mélanges.

**4.** Un procédé selon la revendication 1, 2 ou 3, dans lequel le rapport du détergeant à l'agent chélatant est compris entre 1/1 et 2/1.

**5.** Un procédé selon l'une quelconque des revendications précédentes, dans lequel on a, en outre, un produit choisi parmi des détergents anioniques supplémentaires, des abaisseurs de point de congélation, des aromatisants, des colorants, des parfums, des conservateurs, des adjuvants, des agents antimicrobiens et leurs mélanges.

**6.** Un procédé selon la revendication 5, dans lequel le conservateur est un parabène; ou dans lequel l'aromatisant est la menthe; ou dans lequel l'adjuvant est choisi parmi les phosphates, pyrophosphates, orthophosphates, carbonates solubles dans l'eau et leurs mélanges; ou dans lequel le colorant est un colorant F.D. & C. ou D. & C.; ou dans lequel l'abaisseur de point de congélation est un mélange de glycols en une quantité de 0,5 à 2%; ou dans lequel le détergent anionique supplémentaire est choisi parmi l'ester de l'acide oléique de l'iséthionate de sodium, le N-cyclohexyl-N-palmitoyl-taurate de sodium, le N-(dérivé acide de l'huile de copra)-N-méthyltaurate de sodium, le N-méthyl-N-oléyl-taurate de sodium, les tensio-actifs fluorochimiques ayant la formule:

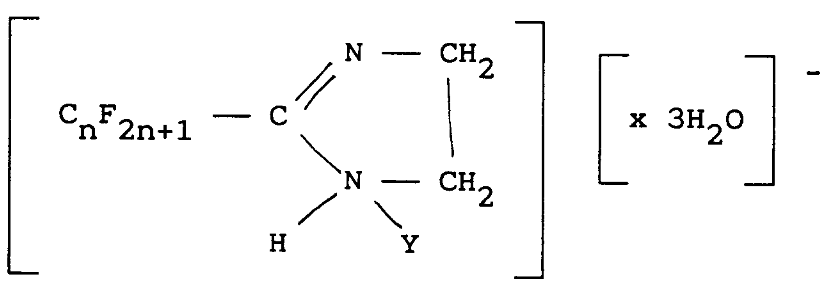

dans laquelle:
- n  est un entier de 3 à 10;
- X  est le brome ou l'iode; et
- Y  est un résidu glycol ayant 3 à 10 atomes de carbone et dérivant par exemple de l'éthylèneglycol ou du propylèneglycol, sous forme d'un alcool éthoxylé ayant 1 à 10 moles d'oxyde d'éthylène ou de propylène,

et leurs mélanges.

**7.** Un procédé de nettoyage d'une prothèse dentaire, comprenant les étapes consistant à:
(a) pulvériser sur la prothèse dentaire une composition nettoyante liquide selon l'une quelconque

des revendications 1 à 6; et
(b) rincer la prothèse dentaire à l'eau.

**8.** Un procédé selon la revendication 7, dans lequel la pulvérisation se fait à une force d'au moins $9{,}8.10^{-3}$ N (un gramme force); ou dans lequel le jet de pulvérisation est appliqué sur une zone d'au moins 3,8 à 6,35 cm (1,5 à 2,5 inch) quand le jet est pulvérisé à partir d'une distance de 5,1 cm (2 inch) de la prothèse dentaire; ou dans lequel la composition nettoyante liquide pour prothèse dentaire peut rester sur la prothèse dentaire pendant 20 à 120 secondes avant rinçage à l'eau.

**Patentansprüche**
**Patentansprüche für folgende Vertragsstaaten : BE, CH, DE, FR, GB, IT, LI, LU, NL, SE**

**1.** Flüssige Reinigungszusammensetzung, welche zur Reinigung von Zahnprothesen geeignet ist, umfassend:
(i) ein sulfatiertes oder sulfoniertes Fettalkoholsalz-Waschmittel, welches einen $C_{10-16}$-Alkylanteil und ein Alkali- oder Erdalkalimetall umfaßt, und welches gegebenenfalls einen aromatischen Anteil umfaßt, welches Waschmittel gegebenenfalls die Formel $ROSO_3M$ aufweist, worin R für ein $C_{10-16}$-Alkyl steht, und M ein wasserlösliches Alkalimetall ist, und das Waschmittel in der Zusammensetzung in einer Menge von 10 % bis 15 %, bezogen auf das Gewicht der Gesamtzusammensetzung, anwesend ist;
(ii) ein Chelatbildner des Aminocarboxylat- oder Organophosphonat-Typus, welches in der Zusammensetzung in einer Menge von 3 % bis 12 %, bezogen auf das Gewicht der Gesamtzusammensetzung, anwesend ist; und
(iii) von 50 % bis 87 % Wasser;
worin die Zusammensetzung keine Carbonsäure, kein Carbonsäuresalz, keine Aminosäure oder kein Aminosäuresalz umfaßt.

**2.** Zusammensetzung nach Anspruch 1, worin das sulfatierte Fettalkoholwaschmittel ausgewählt ist aus Kaliumlaurylsulfat, Magnesiumlaurylsulfat, Natriumlaurylsulfat, Natriumcetylsulfat, Natriumtridecylsulfat, Natrium-7-ethyl-2-methyl-4-undecyl-sulfat, Natriumdodecylbenzolsulfonat, Natriumlaurylsulfonacetat und Mischungen davon.

**3.** Zusammensetzung nach Anspruch 1 oder 2, worin das Chelationsmittel ausgewählt ist aus Tetranatrium-ethylendiamintetraacetat-dihydrat, Trinatrium-ethylendiamintetraacetat, Natrium-ethylendiamintetraacetat-dihydrat, Trinatrium-N-hydroxyethylendiamintriacetat-hydrat, Trinatrium-nitrilotriacetat-monohydrat, Pentanatrium-diethylentriaminpentaacetat, Trinatrium-ethylendiamintetraacetat-trihydrat, dem Natriumsalz der 1-Hydroxy-1,1-diphosphonsäure, dem Kaliumsalz der 1-Hydroxy-1,1-diphosphonsäure und Mischungen davon.

**4.** Zusammensetzung nach Anspruch 1, 2 oder 3, worin das Verhältnis von Waschmittel zu Chelationsmittel im Bereich von 1:1 bis 2:1 liegt.

**5.** Zusammensetzung nach irgendeinem vorhergehenden Anspruch, worin zusätzlich ein Material anwesend ist, welches ausgewählt ist aus zusätzlichen anionischen Waschmitteln, Mitteln zur Gefrierpunktserniedrigung, Geschmacksstoffen, Farbstoffen, Duftstoffen, Konservierungsmitteln, Gerüststoffen, antimikrobiellen Mitteln und Mischungen davon.

**6.** Zusammensetzung nach Anspruch 5, worin das Konservierungsmittel ein Paraben ist; oder worin der Geschmacksstoff Minze ist; oder worin der Gerüststoff ausgewählt ist aus wasserlöslichen Phosphaten, Pyrophosphaten, Orthophosphaten, Carbonaten und Mischungen davon; oder worin der Farbstoff ein F.D. & C. oder D. & C. Farbstoff ist; oder worin das Mittel zur Gefrierpunktserniedrigung eine Glykolmischung in einer Menge von 0,5 % bis 2 % ist; oder worin das zusätzliche anionische Waschmittel ausgewählt ist aus dem Ölsäureester von Natriumisethionat, Natrium-N-cyclohexyl-N-palmitoyl-taurat, Natrium-N-kokossäure-N-methyltaurat, Natrium-N-methyl-N-oleyltaurat, fluorochemischen grenzflächenaktiven Mitteln der Formel:

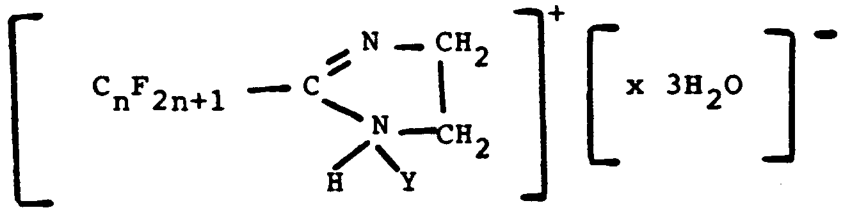

,

worin n eine ganze Zahl von 3 bis 10 ist, X für Brom oder Iod steht, und Y ein Glykolrest mit 3 bis 10 Kohlenstoffatomen ist, welcher beispielsweise von Ethylenglykol oder Propylenglykol als ethoxyliertem Alkohol mit 1 bis 10 Mol Ethylen- oder Propylenoxid stammt, und Mischungen davon.

**7.** Verfahren zur Reinigung einer Zahnprothese umfassend:
(a) Besprühen der Zahnprothese mit einer flüssigen Reinigungszusammensetzung wie in irgendeinem der Ansprüche 1 bis 6 beansprucht und
(b) Abspülen der Zahnprothese mit Wasser.

**8.** Verfahren nach Anspruch 7, worin der Spray eine Kraft von zumindest 9,8 x $10^{-3}$N (1 Gramm Kraft) hat; oder worin der Spray einen Bereich von zumindest 3,8 cm (1,5 Zoll) bis 6,35 cm (2,5 Zoll) besprüht, wenn aus einer Entfernung von 5,1 cm (2 Zoll) von der Zahnprothese gesprüht wird; oder worin die flüssige Zusammensetzung für Zahnprothesen während 20 bis 120 Sekunden vor dem Abspülen mit Wasser auf der Zahnprothese verweilen gelassen wird.

**Patentansprüche für folgenden Vertragsstaat : AT**

**1.** Verfahren zur Herstellung einer flüssigen Reinigungszusammensetzung, welche zur Reinigung von Zahnprothesen geeignet ist, welches Verfahren das Mischen von:
(i) einem sulfatierten oder sulfonierten Fettalkoholsalz-Waschmittel, welches einen $C_{10-16}$-Alkylanteil und ein Alkali- oder Erdalkalimetall umfaßt, und welches gegebenenfalls einen aromatischen Anteil umfaßt, welches Waschmittel gegebenenfalls die Formel $ROSO_3M$ aufweist, worin R für ein $C_{10-6}$-Alkyl steht, und M ein wasserlösliches Alkalimetall ist, und welches Waschmittel in der Zusammensetzung in einer Menge von 10 % bis 15 %, bezogen auf das Gewicht der Gesamtzusammensetzung, anwesend ist;
(ii) einem Chelatbildner des Aminocarboxylat- oder Organophosphonat-Typus, welches in der Zusammensetzung in einer Menge von 3 % bis 12 %, bezogen auf das Gewicht der Gesamtzusammensetzung, anwesend ist; und
(iii) von 50 % bis 87 % Wasser
umfaßt, und worin die Zusammensetzung keine Carbonsäure, kein Carbonsäuresalz, keine Aminosäure oder kein Aminosäuresalz umfaßt.

**2.** Verfahren nach Anspruch 1, worin das sulfatierte Fettalkoholwaschmittel ausgewählt ist aus Kaliumlaurylsulfat, Magnesiumlaurylsulfat, Natriumlaurylsulfat, Natriumcetylsulfat, Natriumtridecylsulfat, Natrium-7-ethyl-2-methyl-4-undecyl-sulfat, Natriumdodecylbenzolsulfonat, Natriumlaurylsulfonacetat und Mischungen davon.

**3.** Verfahren nach Anspruch 1 oder 2, worin das Chelationsmittel ausgewählt ist aus Tetranatrium-ethylendiamintetraacetat-dihydrat, Trinatrium-ethylendiamintetraacetat, Natrium-ethylendiamintetraacetat-dihydrat, Trinatrium-N-hydroxyethylendiamintriacetat-hydrat, Trinatrium-nitrilotriacetat-monohydrat, Pentanatrium-diethylentriaminpentaacetat, Trinatrium-ethylendiamintetraacetat-trihydrat, dem Natriumsalz der 1-Hydroxy-1,1-diphosphonsäure, dem Kaliumsalz der 1-Hydroxy-1,1-diphosphonsäure und Mischungen davon.

**4.** Verfahren nach Anspruch 1, 2 oder 3, worin das Verhältnis von Waschmittel zu Chelationsmittel im Bereich von 1:1 bis 2:1 liegt.

**5.** Verfahren nach irgendeinem vorhergehenden Anspruch, worin zusätzlich ein Material anwesend ist, welches ausgewählt ist aus zusätzlichen anionischen Waschmitteln, Mitteln zur Gefrierpunktserniedrigung, Geschmacksstoffen, Farbstoffen, Duftstoffen, Konservierungsmitteln, Gerüststoffen, antimikrobiellen Mitteln und Mischungen davon.

**6.** Verfahren nach Anspruch 5, worin das Konservierungsmittel ein Paraben ist, oder worin der Geschmacksstoff Minze ist; oder worin der Gerüststoff ausgewählt ist aus wasserlöslichen Phosphaten, Pyrophosphaten, Orthophosphaten, Carbonaten und Mischungen davon; oder worin der Farbstoff ein F.D. & C. oder D. & C. Farbstoff ist; oder worin das Mittel zur Gefrierpunktserniedrigung eine Glykolmischung in einer Menge von 0,5 % bis 2 % ist; oder worin das zusätzliche anionische Waschmittel ausgewählt ist aus dem Ölsäureester von Natriumisethionat, Natrium-N-cyclohexyl-N-palmitoyl-taurat, Natrium-N-kokossäure-N-methyltaurat, Natrium-N-methyl-N-oleyltaurat, fluorochemischen grenzflächenaktiven Mitteln der Formel:

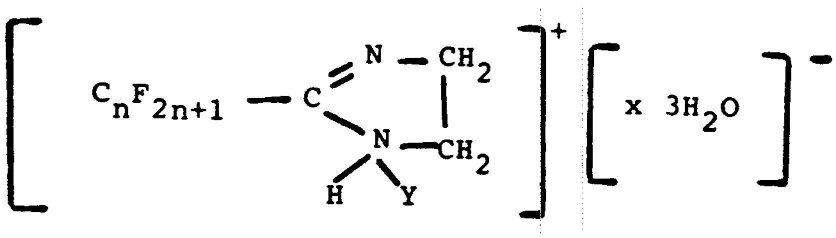

,

worin n eine ganze Zahl von 3 bis 10 ist, X für Brom oder Iod steht, und Y ein Glykolrest mit 3 bis 10 Kohlenstoffatomen ist, welcher beispielsweise von Ethylenglykol oder Propylenglykol als ethoxyliertem Alkohol mit 1 bis 10 Mol Ethylen- oder Propylenoxid stammt, und Mischungen davon.

**7.** Verfahren zur Reinigung einer Zahnprothese umfassend:
(a) Besprühen der Zahnprothese mit einer flüssigen Reinigungszusammensetzung wie in irgendeinem der Ansprüche 1 bis 6 beansprucht und
(b) Abspülen der Zahnprothese mit Wasser.

**8.** Verfahren nach Anspruch 7, worin der Spray eine Kraft von zumindest 9,8 x $10^{-3}$N (1 Gramm Kraft) hat; oder worin der Spray einen Bereich von zumindest 3,8 cm (1,5 Zoll) bis 6,35 cm (2,5 Zoll) besprüht, wenn aus einer Entfernung von 5,1 cm (2 Zoll) von der Zahnprothese gesprüht wird; oder worin die flüssige Zusammensetzung für Zahnprothesen während 20 bis 120 Sekunden vor dem Abspülen mit Wasser auf der Zahnprothese verweilen gelassen wird.

Fig. 1

FOOD STAIN

TILES: COFFEE, TEA, BLUEBERRY AND GRAPE.

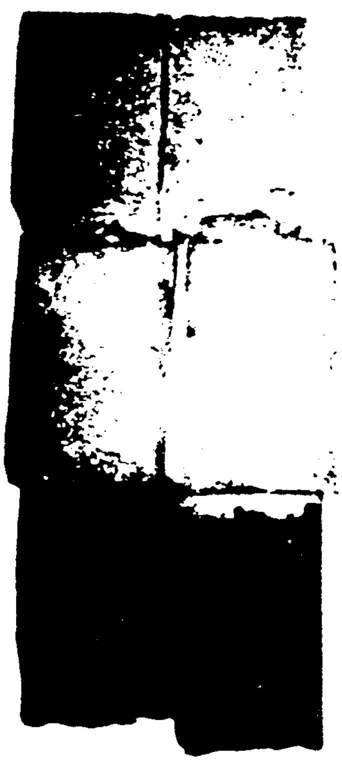

Fig. 2

TOBACCO AND FOOD STAIN TILES: COFFEE, TEA, BLUEBERRY AND GRAPE ON PLAQUE MATRIX

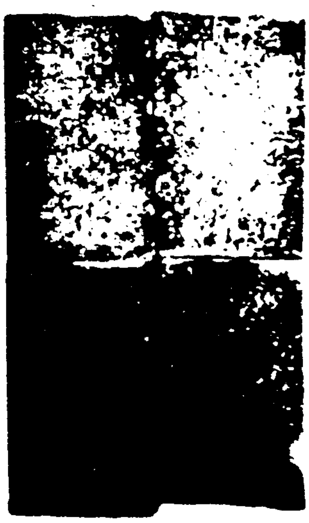

INVENTION COMPOSITION

PRIOR ART 2